# EUROPEAN PATENT SPECIFICATION

(11) **EP 2 248 832 B1**
(45) Date of publication and mention of the grant of the patent: **03.09.2014**
(21) Application number: 08706693.2
(22) Date of filing: 18.02.2008
(51) Int. Cl.: C07K 17/08, C07K 14/535, A61K 47/48, A61K 38/19, A61K 31/04, A61P 31/18, A61P 35/00, A61P 37/00

(54) **A G-CSF CONJUGATE MODIFIED BY WATER-SOLUBLE POLYMER**
DURCH EIN WASSERLÖSLICHES POLYMER MODIFIZIERTES G-CSF-KONJUGAT
CONJUGUÉ DE G-CSF MODIFIÉ PAR UN POLYMÈRE HYDROSOLUBLE

(43) Date of publication of application: 10.11.2010
(73) Proprietor: Jiangsu Hengrui Medicine Co., Ltd., Lianyungang, Jiangsu 222002 (CN)
(72) Inventor: WANG, Ruijun, Lianyungang Jiangsu 222002 (CN); SUN, Changan, Lianyungang Jiangsu 222002 (CN); JIANG, Tao, Lianyungang Jiangsu 222002 (CN); WANG, Yali, Lianyungang Jiangsu 222002 (CN)
(74) Representative: Potter Clarkson LLP
(86) International application number: PCT/CN2008/070320
(87) International publication number: WO 2009/103199

(56) References cited:
- WO-A1-2006/135176
- WO-A2-2005/055946
- WO-A2-2006/063055
- WO-A2-2007/019331
- CN-A- 101 172 161

### SEQUENCE LISTING

<110> JIANGSU HENGRUI MEDICINE CO., LTD.
   <120> A G-CSF conjugate modified by water-soluble polymer
<130> GECCX/P46708EP
<140> EP 08706693.2
   <141> 18-02-08
<150> PCT/CN2008/070320
   <151> 18-02-08
<160> 10
<170> PatentIn version 3.3
<210> 1
   <211> 175
   <212> PRT
   <213>
<400> 1
   Met Thr Pro Leu Gly Pro Ala Ser Ser Leu Pro Gln Ser Phe Leu Leu
   1               5                   10                  15
   Lys Cys Leu Glu Gln Val Arg Lys Ile Gln Gly Asp Gly Ala Ala Leu
               20                  25                  30
   Gln Glu Lys Leu Cys Ala Thr Tyr Lys Leu Cys His Pro Glu Glu Leu
           35                  40                  45
   Val Leu Leu Gly His Ser Leu Gly Ile Pro Trp Ala Pro Leu Ser Ser
       50                  55                  60
   Cys Pro Ser Gln Ala Leu Gln Leu Ala Gly Cys Leu Ser Gln Leu His
   65                  70                  75                  80
   Ser Gly Leu Phe Leu Tyr Gln Gly Leu Leu Gln Ala Leu Glu Gly Ile
                   85                  90                  95
   Ser Pro Glu Leu Gly Pro Thr Leu Asp Thr Leu Gln Leu Asp Val Ala
               100                 105                 110
   Asp Phe Ala Thr Thr Ile Trp Gln Gln Met Glu Glu Leu Gly Met Ala
           115                 120                 125
   Pro Ala Leu Gln Pro Thr Gln Gly Ala Met Pro Ala Phe Ala Ser Ala
       130                 135                 140
   Phe Gln Arg Arg Ala Gly Gly Val Leu Val Ala Ser His Leu Gln Ser
   145                 150                 155                 160
   Phe Leu Glu Val Ser Tyr Arg Val Leu Arg His Leu Ala Gln Pro
                   165                 170                 175
<210> 2
   <211> 174
   <212> PRT
   <213>
<400> 2
   Thr Pro Leu Gly Pro Ala Ser Ser Leu Pro Gln Ser Phe Leu Leu Lys
   1               5                   10                  15
   Cys Leu Glu Gln Val Arg Lys Ile Gln Gly Asp Gly Ala Ala Leu Gln
               20                  25                  30
   Glu Lys Leu Cys Ala Thr Tyr Lys Leu Cys His Pro Glu Glu Leu Val
           35                  40                  45
   Leu Leu Gly His Ser Leu Gly Ile Pro Trp Ala Pro Leu Ser Ser Cys
       50                  55                  60
   Pro Ser Gln Ala Leu Gln Leu Ala Gly Cys Leu Ser Gln Leu His Ser
   65                  70                  75                  80
   Gly Leu Phe Leu Tyr Gln Gly Leu Leu Gln Ala Leu Glu Gly Ile Ser
                   85                  90                  95
   Pro Glu Leu Gly Pro Thr Leu Asp Thr Leu Gln Leu Asp Val Ala Asp
               100                 105                 110
   Phe Ala Thr Thr Ile Trp Gln Gln Met Glu Glu Leu Gly Met Ala Pro
           115                 120                 125
   Ala Leu Gln Pro Thr Gln Gly Ala Met Pro Ala Phe Ala Ser Ala Phe
       130                 135                 140
   Gln Arg Arg Ala Gly Gly Val Leu Val Ala Ser His Leu Gln Ser Phe
   145                 150                 155                 160
   Leu Glu Val Ser Tyr Arg Val Leu Arg His Leu Ala Gln Pro
   165 170
<210> 3
   <211> 175
   <212> PRT
   <213>
<400> 3
   Met Thr Pro Leu Gly Pro Ala Ser Ser Leu Pro Gln Ser Phe Leu Leu
   1               5                   10                  15
   Lys Ser Leu Glu Gln Val Arg Lys Ile Gln Gly Asp Gly Ala Ala Leu
               20                  25                  30
   Gln Glu Lys Leu Cys Ala Thr Tyr Lys Leu Cys His Pro Glu Glu Leu
           35                  40                  45
   Val Leu Leu Gly His Ser Leu Gly Ile Pro Trp Ala Pro Leu Ser Ser
       50                  55                  60
   Cys Pro Ser Gln Ala Leu Gln Leu Ala Gly Cys Leu Ser Gln Leu His
   65                  70                  75                  80
   Ser Gly Leu Phe Leu Tyr Gln Gly Leu Leu Gln Ala Leu Glu Gly Ile
                   85                  90                  95
   Ser Pro Glu Leu Gly Pro Thr Leu Asp Thr Leu Gln Leu Asp Val Ala
               100                 105                 110
   Asp Phe Ala Thr Thr Ile Trp Gln Gln Met Glu Glu Leu Gly Met Ala
           115                 120                 125
   Pro Ala Leu Gln Pro Thr Gln Gly Ala Met Pro Ala Phe Ala Ser Ala
       130                 135                 140
   Phe Gln Arg Arg Ala Gly Gly Val Leu Val Ala Ser His Leu Gln Ser
   145                 150                 155                 160
   Phe Leu Glu Val Ser Tyr Arg Val Leu Arg His Leu Ala Gln Pro
                   165                 170                 175
<210> 4
   <211> 174
   <212> PRT
   <213>
<400> 4
   Thr Pro Leu Gly Pro Ala Ser Ser Leu Pro Gln Ser Phe Leu Leu Lys
   1 5 10 15
   Ser Leu Glu Gln Val Arg Lys Ile Gln Gly Asp Gly Ala Ala Leu Gln
   20 25 30
   Glu Lys Leu Cys Ala Thr Tyr Lys Leu Cys His Pro Glu Glu Leu Val
   35 40 45
   Leu Leu Gly His Ser Leu Gly Ile Pro Trp Ala Pro Leu Ser Ser Cys
   50 55 60
   Pro Ser Gln Ala Leu Gln Leu Ala Gly Cys Leu Ser Gln Leu His Ser
   65 70 75 80
   Gly Leu Phe Leu Tyr Gln Gly Leu Leu Gln Ala Leu Glu Gly Ile Ser
   85 90 95
   Pro Glu Leu Gly Pro Thr Leu Asp Thr Leu Gln Leu Asp Val Ala Asp
   100 105 110
   Phe Ala Thr Thr Ile Trp Gln Gln Met Glu Glu Leu Gly Met Ala Pro
   115 120 125
   Ala Leu Gln Pro Thr Gln Gly Ala Met Pro Ala Phe Ala Ser Ala Phe
   130 135 140
   Gln Arg Arg Ala Gly Gly Val Leu Val Ala Ser His Leu Gln Ser Phe
   145 150 155 160
   Leu Glu Val Ser Tyr Arg Val Leu Arg His Leu Ala Gln Pro
   165 170
<210> 5
   <211> 175
   <212> PRT
   <213>
<400> 5
   Trp Thr Pro Leu Gly Pro Ala Ser Ser Leu Pro Gln Ser Phe Leu Leu
   1 5 10 15
   Lys Cys Leu Glu Gln Val Arg Lys Ile Gln Gly Asp Gly Ala Ala Leu
   20 25 30
   Gln Glu Lys Leu Cys Ala Thr Tyr Lys Leu Cys His Pro Glu Glu Leu
   35 40 45
   Val Leu Leu Gly His Ser Leu Gly Ile Pro Trp Ala Pro Leu Ser Ser
   50 55 60
   Cys Pro Ser Gln Ala Leu Gln Leu Ala Gly Cys Leu Ser Gln Leu His
   65 70 75 80
   Ser Gly Leu Phe Leu Tyr Gln Gly Leu Leu Gln Ala Leu Glu Gly Ile
   85 90 95
   Ser Pro Glu Leu Gly Pro Thr Leu Asp Thr Leu Gln Leu Asp Val Ala
   100 105 110
   Asp Phe Ala Thr Thr Ile Trp Gln Gln Met Glu Glu Leu Gly Met Ala
   115 120 125
   Pro Ala Leu Gln Pro Thr Gln Gly Ala Met Pro Ala Phe Ala Ser Ala
   130 135 140
   Phe Gln Arg Arg Ala Gly Gly Val Leu Val Ala Ser His Leu Gln Ser
   145 150 155 160
   Phe Leu Glu Val Ser Tyr Arg Val Leu Arg His Leu Ala Gln Pro
   165 170 175
<210> 6
   <211> 175
   <212> PRT
   <213>
<400> 6
   Trp Thr Pro Leu Gly Pro Ala Ser Ser Leu Pro Gln Ser Phe Leu Leu
   1               5                   10                  15
   Lys Ser Leu Glu Gln Val Arg Lys Ile Gln Gly Asp Gly Ala Ala Leu
               20                  25                  30
   Gln Glu Lys Leu Cys Ala Thr Tyr Lys Leu Cys His Pro Glu Glu Leu
           35                  40                  45
   Val Leu Leu Gly His Ser Leu Gly Ile Pro Trp Ala Pro Leu Ser Ser
       50                  55                  60
   Cys Pro Ser Gln Ala Leu Gln Leu Ala Gly Cys Leu Ser Gln Leu His
   65                  70                  75                  80
   Ser Gly Leu Phe Leu Tyr Gln Gly Leu Leu Gln Ala Leu Glu Gly Ile
                   85                  90                  95
   Ser Pro Glu Leu Gly Pro Thr Leu Asp Thr Leu Gln Leu Asp Val Ala
               100                 105                 110
   Asp Phe Ala Thr Thr Ile Trp Gln Gln Met Glu Glu Leu Gly Met Ala
           115                 120                 125
   Pro Ala Leu Gln Pro Thr Gln Gly Ala Met Pro Ala Phe Ala Ser Ala
       130                 135                 140
   Phe Gln Arg Arg Ala Gly Gly Val Leu Val Ala Ser His Leu Gln Ser
   145                 150                 155                 160
   Phe Leu Glu Val Ser Tyr Arg Val Leu Arg His Leu Ala Gln Pro
                   165                 170                 175
<210> 7
   <211> 175
   <212> PRT
   <213>
<400> 7
   Asp Thr Pro Leu Gly Pro Ala Ser Ser Leu Pro Gln Ser Phe Leu Leu
   1               5                   10                  15
   Lys Cys Leu Glu Gln Val Arg Lys Ile Gln Gly Asp Gly Ala Ala Leu
               20                  25                  30
   Gln Glu Lys Leu Cys Ala Thr Tyr Lys Leu Cys His Pro Glu Glu Leu
           35                  40                  45
   Val Leu Leu Gly His Ser Leu Gly Ile Pro Trp Ala Pro Leu Ser Ser
       50                  55                  60
   Cys Pro Ser Gln Ala Leu Gln Leu Ala Gly Cys Leu Ser Gln Leu His
   65                  70                  75                  80
   Ser Gly Leu Phe Leu Tyr Gln Gly Leu Leu Gln Ala Leu Glu Gly Ile
                   85                  90                  95
   Ser Pro Glu Leu Gly Pro Thr Leu Asp Thr Leu Gln Leu Asp Val Ala
               100                 105                 110
   Asp Phe Ala Thr Thr Ile Trp Gln Gln Met Glu Glu Leu Gly Met Ala
           115                 120                 125
   Pro Ala Leu Gln Pro Thr Gln Gly Ala Met Pro Ala Phe Ala Ser Ala
       130                 135                 140
   Phe Gln Arg Arg Ala Gly Gly Val Leu Val Ala Ser His Leu Gln Ser
   145                 150                 155                 160
   Phe Leu Glu Val Ser Tyr Arg Val Leu Arg His Leu Ala Gln Pro
                   165                 170                 175
<210> 8
   <211> 175
   <212> PRT
   <213>
<400> 8
   Asp Thr Pro Leu Gly Pro Ala Ser Ser Leu Pro Gln Ser Phe Leu Leu
   1 5 10 15
   Lys Ser Leu Glu Gln Val Arg Lys Ile Gln Gly Asp Gly Ala Ala Leu
   20 25 30
   Gln Glu Lys Leu Cys Ala Thr Tyr Lys Leu Cys His Pro Glu Glu Leu
   35 40 45
   Val Leu Leu Gly His Ser Leu Gly Ile Pro Trp Ala Pro Leu Ser Ser
   50 55 60
   Cys Pro Ser Gln Ala Leu Gln Leu Ala Gly Cys Leu Ser Gln Leu His
   65 70 75 80
   Ser Gly Leu Phe Leu Tyr Gln Gly Leu Leu Gln Ala Leu Glu Gly Ile
   85 90 95
   Ser Pro Glu Leu Gly Pro Thr Leu Asp Thr Leu Gln Leu Asp Val Ala
   100 105 110
   Asp Phe Ala Thr Thr Ile Trp Gln Gln Met Glu Glu Leu Gly Met Ala
   115 120 125
   Pro Ala Leu Gln Pro Thr Gln Gly Ala Met Pro Ala Phe Ala Ser Ala
   130 135 140
   Phe Gln Arg Arg Ala Gly Gly Val Leu Val Ala Ser His Leu Gln Ser
   145 150 155 160
   Phe Leu Glu Val Ser Tyr Arg Val Leu Arg His Leu Ala Gln Pro
   165 170 175
<210> 9
   <211> 175
   <212> PRT
   <213>
<400> 9
   Glu Thr Pro Leu Gly Pro Ala Ser Ser Leu Pro Gln Ser Phe Leu Leu
   1 5 10 15
   Lys Cys Leu Glu Gln Val Arg Lys Ile Gln Gly Asp Gly Ala Ala Leu
   20 25 30
   Gln Glu Lys Leu Cys Ala Thr Tyr Lys Leu Cys His Pro Glu Glu Leu
   35 40 45
   Val Leu Leu Gly His Ser Leu Gly Ile Pro Trp Ala Pro Leu Ser Ser
   50 55 60
   Cys Pro Ser Gln Ala Leu Gln Leu Ala Gly Cys Leu Ser Gln Leu His
   65 70 75 80
   Ser Gly Leu Phe Leu Tyr Gln Gly Leu Leu Gln Ala Leu Glu Gly Ile
   85 90 95
   Ser Pro Glu Leu Gly Pro Thr Leu Asp Thr Leu Gln Leu Asp Val Ala
   100 105 110
   Asp Phe Ala Thr Thr Ile Trp Gln Gln Met Glu Glu Leu Gly Met Ala
   115 120 125
   Pro Ala Leu Gln Pro Thr Gln Gly Ala Met Pro Ala Phe Ala Ser Ala
   130 135 140
   Phe Gln Arg Arg Ala Gly Gly Val Leu Val Ala Ser His Leu Gln Ser
   145 150 155 160
   Phe Leu Glu Val Ser Tyr Arg Val Leu Arg His Leu Ala Gln Pro
   165 170 175
<210> 10
   <211> 175
   <212> PRT
   <213>
<400> 10
   Glu Thr Pro Leu Gly Pro Ala Ser Ser Leu Pro Gln Ser Phe Leu Leu
   1               5                   10                  15
   Lys Ser Leu Glu Gln Val Arg Lys Ile Gln Gly Asp Gly Ala Ala Leu
               20                  25                  30
   Gln Glu Lys Leu Cys Ala Thr Tyr Lys Leu Cys His Pro Glu Glu Leu
           35                  40                  45
   Val Leu Leu Gly His Ser Leu Gly Ile Pro Trp Ala Pro Leu Ser Ser
       50                  55                  60
   Cys Pro Ser Gln Ala Leu Gln Leu Ala Gly Cys Leu Ser Gln Leu His
   65                  70                  75                  80
   Ser Gly Leu Phe Leu Tyr Gln Gly Leu Leu Gln Ala Leu Glu Gly Ile
                   85                  90                  95
   Ser Pro Glu Leu Gly Pro Thr Leu Asp Thr Leu Gln Leu Asp Val Ala
               100                 105                 110
   Asp Phe Ala Thr Thr Ile Trp Gln Gln Met Glu Glu Leu Gly Met Ala
           115                 120                 125
   Pro Ala Leu Gln Pro Thr Gln Gly Ala Met Pro Ala Phe Ala Ser Ala
       130                 135                 140
   Phe Gln Arg Arg Ala Gly Gly Val Leu Val Ala Ser His Leu Gln Ser
   145                 150                 155                 160
   Phe Leu Glu Val Ser Tyr Arg Val Leu Arg His Leu Ala Gln Pro
                   165                 170                 175

## Description

### FIELD OF THE INVENTION

This invention relates to a G-CSF conjugate modified by water-soluble polymer having formula (I): water-soluble polymer-linking group-N-terminal of G-CSF, or pharmaceutically acceptable salts thereof, the preparation method thereof and the pharmaceutical composition comprising the same. In particular, it relates to a G-CSF conjugate of general formula (II).

### BACKGROUND OF THE INVENTION

Granulocyte colony stimulating factor (G-CSF) is produced by mononuclear cells and fibroblast cells. It can stimulate granulocyte to form colony, and has stimulating effect on neutrophil. By combining with membrane receptors of the target cell, G-CSF mainly stimulates hematopoiesis of granulocyte and also promotes multipotential hematopoietic stem cells to enter cell cycle; promotes proliferation, differentiation and maturation of myeloid hematopoietic progenitors, and drives the release of neutrophils into blood, meanwhile increases the number of peripheral neutrophils and improves their function, such as phagocytosis, antibody-dependent cellular cytotoxic activity against tumor cells, etc. [Metcalf, Blood 67:257(1986); Yan et. al., Blood 84(3): 795-799(1994); Bensinger, et. al., Blood 81(11): 3158-3163(1993); Neben, et. al., Blood 81(7): 1960-1967(1993)]. Therefore, recombinant granulocyte colony stimulating factor is commonly used in cancer patients subjected to radiotherapy or chemotherapy, and in leukemia patients after bone marrow transplantation as an adjuvant treatment.

Human G-CSFs commonly used on market are Neupogen and Neutrogin, and human G-CSF derivative, i.e. Neu-wp. G-CSF derivatives or variant proteins have also been reported in a large number of literatures [such as US5581476, US5214132, US5362853, US4904584]. These variant proteins have multiple amino acid substitutions which have been designed to explore G-CSF which is more stable, more active and more suitable for clinical use.

Commercially available recombinant human G-CSF needs to be injected frequently and is difficult to achieve good clinical effect because of its poor bioavailability, short half-life in human body and vulnerability to proteases in vivo. The research shows that the possibility of the proteins having therapeutic applications to become drugs is greatly increased after it is modified by polyethylene glycol to become a PEG - protein conjugate. This kind of PEGylated proteins have been fully applied in clinical practice [such as Katre, Advanced Drug Delivery Systems, 10:91(1993); Inada; et. al.; J. Bioact and Compatible Polymers; 5:343(1990)]. Polyethylene glycol-modified protein conjugates not only have better physical and chemical stability, but also better resistance to protease in vivo; In addition, as the molecular weight of the conjugates increases, the half-life of the conjugates in vivo is extended. The toxicity will be reduced because of lower possibility to produce antibodies in vivo and the reduced distribution volume of the conjugates as comparing with original proteins. PEG-modified G-CSF or G-CSF variant proteins have been disclosed in numerous literatures, such as EP0335423, EP0401384, US5824778, US5985265, WO0044785, WO2001051510, US5824784, etc. Specifically, among the polyethylene glycol modified G-CSF conjugates disclosed by patent US5985265, the conjugates modified at the N terminal of G-CSF have the best biological activities both in vitro and in vivo. But the amino group selectivity is poor when polyethylene glycol modification is carried out through acylation reaction, so a mixture form the modification at various positions (amino group) of G-CSF by polyethylene glycol is generally obtained. Separation and purification are required to obtain each monomer with low yield and are difficult to apply in industrial manufacture. In patent US5824784, polyethylene glycol aldehyde with big molecular weight was used to modify G-CSF directly. With strictly controlling the pH of the reaction, relatively specific N-terminal PEG-modified conjugates can be obtained. Undeniably, however, the reaction with high selectivity is achieved by the different Pka between side chain amino group and N-terminal amino group, which is very difficult to control in the production scale. In addition, because the variable content of aldehydes in the polyethylene glycol aldehyde with big molecular weight in each batch, it is difficult to control the feed ratio between macromolecular aldehydes and the proteins, which will definitely affects the reaction yield and production costs. Meanwhile, the different biological activity of the conjugates produced by macromolecular aldehydes coupled with different amino groups will affect the homogeneity and activity of the final product too.

WO 2005/0055946 (D1) describes glycopegylated GCSF.

WO 2007/019331 (D2) describes conjugates of a GCSF moiety and a polymer.

### DESCRIPTION OF THE INVENTION

In view of the deficiencies of prior art, the purpose of the present invention is to provide a G-CSF conjugate having a new structure, general formula (II), or pharmaceutically acceptable salts thereof. Another purpose of the present invention is to provide a method of preparing the conjugate of general formula (II) or pharmaceutically acceptable salts thereof. Another purpose of the present invention is to provide a pharmaceutical composition containing such conjugates or pharmaceutically acceptable salts and use thereof.

This invention relates to a conjugate of general formula (I) or pharmaceutically acceptable salts thereof.

G-CSF is selected from natural G-CSF, recombinant G-CSF or gene mutation products with the function of G-CSF, preferably G-CSF is G-CSF derivatives set forth in SEQ ID NO: 1-10, more preferably G-CSF is naturally occurring human G-CSF sequence or Met-G-CSF set forth in SEQ ID NO: 1. 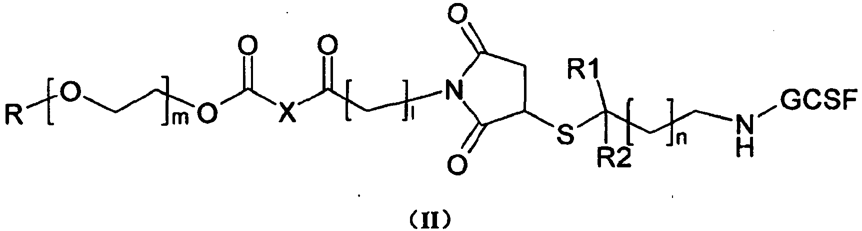 wherein,
R is selected from C₁₋₄ linear or branched alkyl, preferably R is methyl or ethyl, more preferably R is methyl;
R₁ and R₂ are each independently selected from hydrogen or C₁₋₄ linear or branched alkyl; preferably R₁, R₂ are hydrogen, methyl or ethyl;
X is selected from O, S, NH, 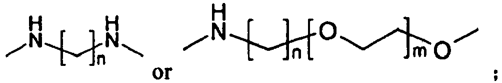
1 is an integer selected from 1-20, preferably 1 is an integer selected from 1-10, more preferably 1 is an integer selected from 1-5;
m is an integer selected from 50-2500, preferably m is an integer selected from 100-1000;
n is an integer selected from 1-20, preferably n is an integer selected from 1-10, more preferably n is an integer selected from 1-5;
G-CSF is selected from natural G-CSF, recombinant G-CSF or gene mutation products with the function of G-CSF.

Further, this invention relates to the foregoing conjugate of general formula (II) or pharmaceutical salts thereof, wherein G-CSF has a naturally occurring human G-CSF sequence.

Further, this invention relates to the foregoing conjugate of general formula (II) or pharmaceutical salts thereof, wherein G-CSF is Met-G-CSF (SEQ ID NO: 1).

The foregoing conjugates of general formula (II) include: 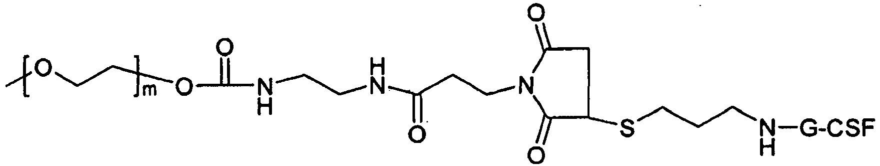 m is an integer selected from 400-500; 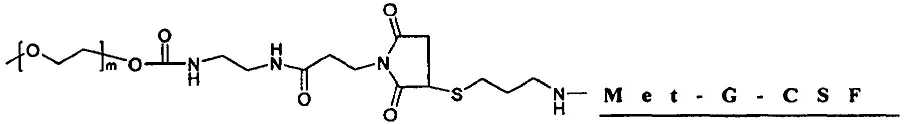 m is an integer selected from 400-500;

Further, the conjugates of the general formula (II) can react with acids to form salts. The acids used are selected from organic acids or inorganic acids, wherein the organic acids are selected from acetic acid, trifluoroacetic acid, propionic acid, butyric acid, maleic acid or p-toluenesulfonic acid or mixtures thereof. Preferably, organic acids are acetic acid, trifluoroacetic acid. Inorganic acids are selected from hydrochloric acid, sulfuric acid, phosphoric acid or sulfonic acid or mixtures thereof. Preferably inorganic acid is hydrochloric acid.

On the other hand, this invention provides a method for preparation of the conjugates of general formula (II) including the following steps of:
1) reacting the compound of general formular (III) with N-terminal amino group of G-CSF through reductive amination to obtain the compound of general formula (IV): 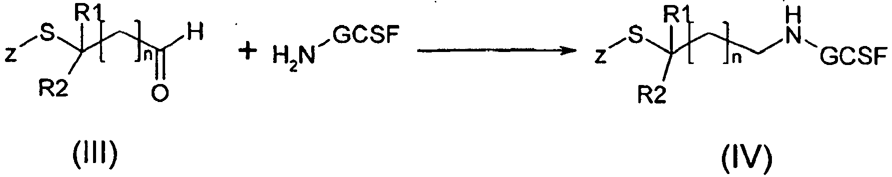
2) removing the protecting group of thiol group from the compound of general formula (IV) to obtain the compound of general formula (V): 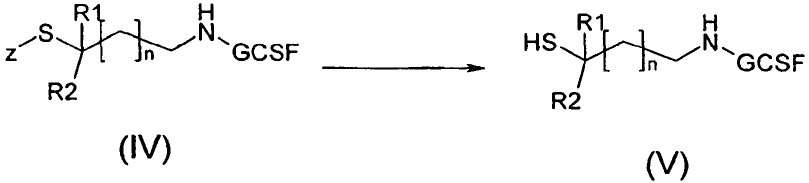
3) reacting the compound of general formula (V) with mPEG-MAL through Michael addition to obtain the conjugate of general formula (II) : 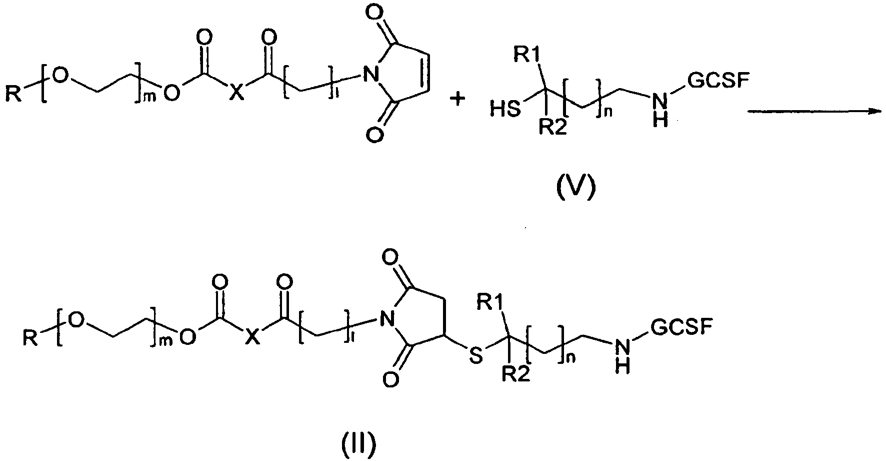 wherein,
   R, R1, R2, G-CSF, X, 1, m, n are defined as above;
   Z is mercapto-protective group selected from formyl, acetyl, propionyl, trityl or tert-butyl group, preferably Z is acetyl group.

Further, this invention also relates to the use of the conjugate or pharmaceutical salts thereof provided by this invention in the preparation of the medicaments treating leukopenia caused by radiotherapy or chemotherapy, AIDS and other immune deficiency diseases, bacterial infections.

On the other hand, this invention provides a pharmaceutical composition containing a pharmaceutically effective amount of the conjugate or pharmaceutically acceptable salts thereof provided by this invention and pharmaceutically acceptable carriers.

Further, this invention also relates to the use of the said pharmaceutical compositions in the preparation of the medicaments treating leukopenia caused by radiotherapy or chemotherapy, AIDS and other immune deficiency diseases, bacterial infections.

This invention discloses a new G-CSF conjugate modified by PEG and a new preparation method thereof. Compared with traditional conjugates and the preparation methods thereof, the following differences are present:
Firstly, introducing linking group between PEG and G-CSF can ensure specificity of the reaction at N-terminal of the protein, because the protein modified with small molecules at its N-terminal can be easily separated and purified, ensuring the specificity of the modified site;
Secondly, since the presence of thiol group in the linking group, controlling pH of the reaction system will ensure Michael addition reaction having high specificity to be carried out;

In addition, because of the existence of the linking group, other functional groups may also join in, such as the introduction of imido group, ester group, which provides preconditions for in vivo release of G-CSF from the conjugates.

Conjugates of the present invention or pharmaceutically acceptable salts thereof have physiological activity of natural human G-CSF, and have longer in vivo circulating half-life and better granulocyte colony-stimulating factor activity than G-CSF.

Specifically, the structure of the conjugate disclosed by the present invention is shown as (II): 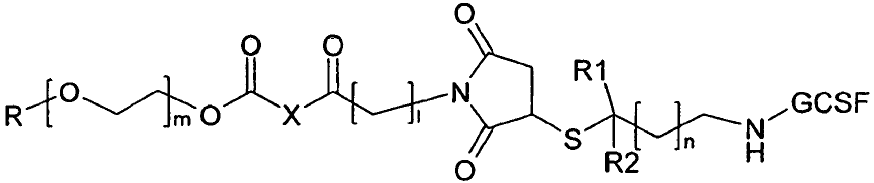 wherein,
R is selected from C₁₋₄ linear or branched alkyl, preferably R is methyl or ethyl, more preferably R is methyl;
R1 and R2 are independently selected from hydrogen or C₁₋₄ linear or branched alkyl, preferably R1, R2 are hydrogen, methyl or ethyl;
X is selected from O, S, NH, 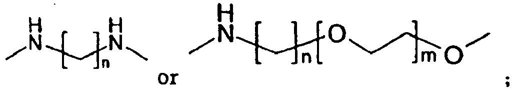
1 is an integer selected from 1-20, preferably 1 is an integer selected froml-10, more preferably 1 is an integer selected from 1-5;
m is an integer selected from 50-2500, preferably m is an integer selected from 100-1000;
n is an integer selected from 1-20, preferably n is an integer selected from 1-5;.
G-CSF is selected from natural G-CSF, recombinant G-CSF or gene mutation products with the function of G-CSF.

When X is O, S, there is an ester bond susceptible to hydrolysis in vivo by the esterase in the linking group between PEG and G-CSF. At this time, G-CSF in its free form can interact with its receptor to exhibit biological activity;
When X is NH, 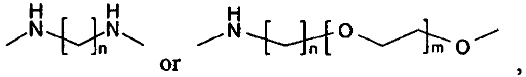 the biological activity is mainly achieved by the conjugate per se. Of course, because imido bond also has the ability to release G-CSF through hydrolysis in vivo, the possibility that the free G-CSF works can not be ruled out.

In the methods of preparation disclosed by the present invention, firstly the intermediate (IV) is obtained by reacting the linking group containing thiol group with N-terminal amino group of G-CSF through reductive amination. In fact, the linking group and the amino group of G-CSF can be connected by a variety of reaction methods (such as reaction of various activated esters with amino group). But taking into account the high selectivity of the reaction between aldehyde group and N terminal amino group, the linker is introduced by reacting the aldehyde group with the N terminal amino group through reductive amination in the present invention. Reducing agent used in the present invention is selected from various reducing agents well-known in the art. Preferably the reducing agents are sodium cyanoborohydride, triacetoxy borohydride.

In the preparation of intermediate (V), different reaction conditions are chosen according to different thiol protecting groups. When the protecting group is trityl or tert-butyl, acidic conditions (such as trifluoroacetic acid, hydrochloric acid, methane sulfonic acid, etc.) are used to remove the protecting group. When the protecting group is acetyl, propionyl, etc., various methods, reagents which are well-known by the skillful person in the art are used to remove the protecting group, preferably hydroxylamine hydrochloride is used to remove the protecting group in pH 5-7.

In the preparation of the compound of general formula (II), the reaction is carried out by controlling pH and using the classical Michael addition. The said reaction has a good selectivity up to more than 99%. After the reaction, the reaction product can be separated and purified by reversed-phase high-performance liquid chromatography (RP-HPLC), ion exchange column or gel column.

In the present invention, "C₁₋₄ alkyl" means linear or branched alkyl, such as methyl, ethyl, propyl, isopropyl, butyl group and so on.

In the present invention, G-CSF is preferably selected from human G-CSF. The absence of variations in internal sequence of this protein can reduce the antigenicity of the protein in vivo and maximally reduce the formation of neutralizing antibodies in vivo to enhance drug efficacy. G-CSF and its variant proteins, including but not limited to Met-G-CSF, Trp-G-CSF, Asp-G-CSF, Glu-G-CSF, preferably Met-G-CSF (for its sequence, see SEQ ID NO: 1) which is expressed by *E. coli.*, can be obtained by using traditional methods of gene expression reported in the literatures.

The compound obtained by the present invention is administrated in dosage units. The said dosage units can be better expressed as the pharmaceutical composition mixed by the active compounds and pharmaceutical excipients in formulation form.

In the pharmaceutical composition provided by the present invention, conjugates of general formula (II) or pharmaceutically acceptably salts thereof are active compounds. The pharmaceutical composition provided by the present invention can be used in adjuvant therapy for cancer patients subjected to chemotherapy or radiotherapy and for patients subjected to bone marrow transplantation in order to prevent the infections due to reduction of immunity. It can also be used for treating patients of chronic or relative leucopenia, for treating patients of acute myeloid leukemia, AIDS or other immune deficiency diseases and also for treating fungal, especially for infection by systemic or invasive candidiasis.

The administration dosage of the conjugates of general formula (II) or pharmaceutically acceptable salts thereof is 5-500ug/kg, wherein "ug" refers to the unit of the conjugates of general formula (II) or pharmaceutically acceptable salts thereof, "kg" refers to the unit of mammalian body weight. It is known that the administration dosage and frequency depend on many factors, such as the patient's sex, age, the type of the diseases needed for prevention or treatment in the patient.

Various formulation forms can be prepared by mixing the conjugates or the pharmaceutically accepted salts thereof provided by the present invention with conventional pharmaceutical carriers, which can be administrated by different ways such as subcutaneous, intramuscular, intravenous administration.

### Description of the Figures

Figure 1 shows MALDI-TOF-TOF diagram of compound 1 provided by the present invention;
Figure 2 shows MALDI-TOF-TOF diagram of compound 2 provided by the present invention;
Figure 3 shows the effect of PEG-G-CSF, G-CSF on peripheral white blood cell count of mice treated by cyclophosphamide;
Figure 4 shows the effect of PEG-G-CSF, G-CSF on peripheral blood erythrocyte count of mice treated by cyclophosphamide;
Figure 5 shows the effect of PEG-G-CSF, G-CSF on peripheral blood platelet count of mice treated by cyclophosphamide.

### PREFERRED EMBODIMENTS

For a more detailed description of the present invention, the following examples are provided.

### Example 1

### Reductive amination of Met-G-CSF

| | |
|---|---|
| Met-G-CSF (pH=5.0) : | 40mg/140 ml |
| 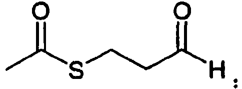 | 7.0 mg |
| Sodium cyanoborohydride: | 176 mg |

The original solution of Met-G-CSF was dialysed against 0.1M acetic acid / sodium acetate solution, from which 140 ml solution containing 40 mg proteins was taken out. Small molecule aldehyde (7.0 mg) was taken out and dissolved in acetonitrile (300ml), which was added into protein solution, then sodium cyanoborohydride (176 mg) was added in and reaction was carried out for 3 hours at room temperature by stirring.

The reaction solution was dialysed against 0.1M of PBS solution (pH = 6.2) containing 2mM EDTA at 4°C.

### Example 2

### De-protection of protecting group on thiol group - acetyl group

10ml prepared 0.1M Hydroxylamine hydrochloride (pH = 6.3) was added into the protein solution prepared by **Example 1** to make the concentration of Hydroxylamine hydrochloride to 50mM. The reaction was carried out by stirring for 30 minutes at room temperature to free the thiol group by removing acetyl group.

### Example 3

### Preparation of mPEG-Met-G-CSF (39KD)

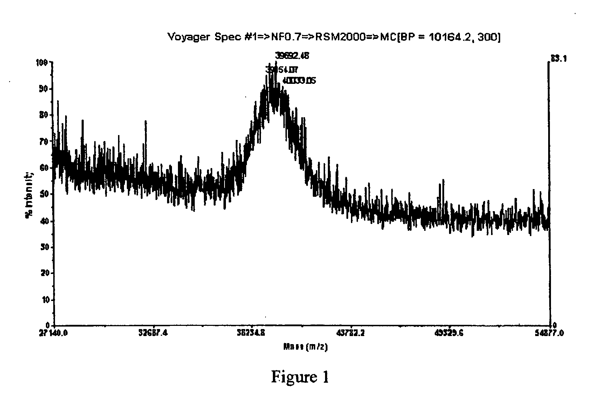

mPEG-MAL(400 mg, 20 KD) was added into the protein solution prepared by Example 2, and reaction was carried out by stirring for 60 minutes at room temperature. The purification was performed after HPLC control shown the reaction was finished. The compound obtained was compound 1 of the present invention. T he structure was confirmed by MALDI -TOF-TOF as shown in Figure 1.

### Control conditions:

Column: Jupiter C4, 5u, 300Å, 150*4.6
Mobile phase: A: 0.05%TFA/H₂O B: 0.05%TFA/CH₃CN

### Gradient conditions:

| | 0' | 30' | 35' | 36' |
|---|---|---|---|---|
| A | 60% | 20% | 20% | 60% |
| B | 40% | 80% | 80% | 40% |

### Conditions for purification:

The column (1.6 cm x 12 cm, the volume was about 24 ml, flowing rate: 4 ml/min) was filled with SP Sepharose HP,

Pre-treatment of the column: The column was washed with a volume of the 5 times volume of the column of 0.5M NaOH solution; and then was washed to neutral pH with purified water; finally the column was balanced with 20 mM HAc / NaAc, pH4.0, the volume used to balance was 5 times of the volume of the column.

Loading the sample: the sample desalted was sucked into the column directly using a pump;
Elution: firstly the column was eluted with 20 mM HAc / NaAc, pH 4.0 whose volume was 10 times of the volume of the column to remove excessive PEG;

Then the salt gradient from 0 to 50% was set with the running time of 50 min at 4 ml/min. The impurities, products and unreacted proteins were eluted and collected in turn.

### Example 4

### Preparation of mPEG-Met-G-CSF (59KD)

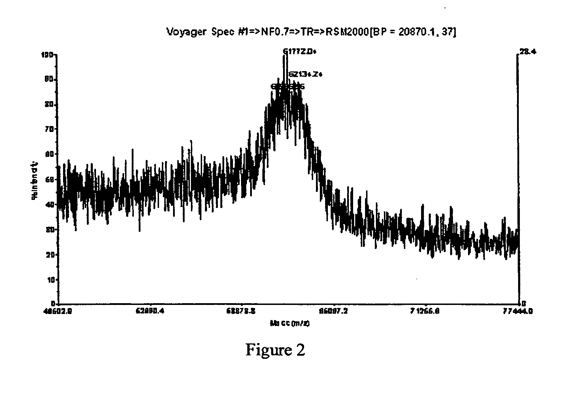

The method of preparation and purification is the same as in **Example 3,** except that mPEG-MAL (400 mg, 20 KD) was replaced with mPEG-MAL (800 mg, 40 KD). The compound obtained is compound 2 of the present invention, whose MALDI-TOF-TOF is shown in Figure 2.

### Example 5

### Preparation of the injectable solution of mPEG-Met-G-CSF (39KD) i.e. compound 1

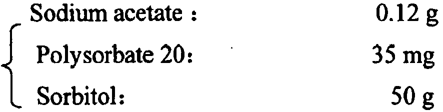

### Compound 1: 10 g

In the aseptic room for preparation, sodium acetate (0.12 g), polysorbate 20 (35 mg), sorbitol (50 g) were weighted and dissolved in injectable water (1000 ml) under stirring. Compound 1 (10 g) was added in and stirred to uniform, and injectable water was added to a final volume of 3000 ml. A fter filtered with 0.22 µm microporous membrane, the solution was packed and plugged with a sterilized stopper and capped.

### Test1

### The comparison of the effect between PEG-G-CSF and G-CSF on increasing the peripheral white blood cell count

Note: PEG-G-CSF refers to compounds 1 of the present invention.

### 1. Purpose of the test

Evaluate and compare the effect of PEG-G-CSF and G-CSF on increasing the peripheral white blood cell count of the mice treated by cyclophosphamide.

### 2. Materials and methods:

PEG-G-CSF, G-CSF and cyclophosphamide (CTX) were provided by Jiangsu Hansoh Pharmaceutical Co., Ltd. and diluted with saline before using.

Female Kunming mice were purchased from Shanghai Experimental Animal Center of Chinese Academy of Sciences, weighting 18-22g with the number of 10 animals in each group.

After the animals adapt to environment, cyclophosphamide was administrated by intraperitoneal injection, and PEG-G-CSF, G-CSF were administrated by subcutaneous injection the next day. PEG -G-CSF was subcutaneously injected at 0.5, 1.0 mg / kg once; G-CSF was subcutaneously injected once per day for 4 consecutive days with the dosage of 0.1, 0.2 mg / kg. A fter the administration, the mice were killed by cervical dislocation and the blood cells were counted with ABC automatic blood cell counter.

### 3. Results

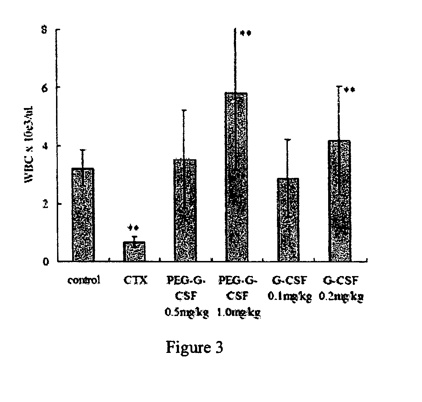

Intraperitoneal injection of CTX significantly decreases counting for peripheral white blood cell, blood erythrocyte, platelet (average P <0.01 compared with the control, Figure 3-5), indicating that CTX is a strong inhibitor of bone marrow.

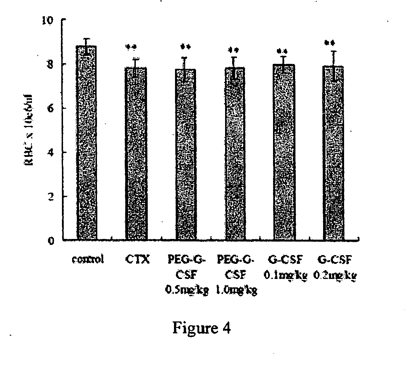

A single subcutaneous injection of PEG-G-CSF to the mice treated by cyclophosphamide increases the peripheral white blood cell count. W hen 0.5mg/kg is administered, the white blood cell count returns to normal level. Whe n 1.0mg/kg is administrated, the peripheral white blood cell count is even higher than normal level (P <0.01 compared with the control, Figure 3). However, PEG-G-CSF has no obvious increasing effect on peripheral blood erythrocyte count or platelet count (Figure 4, 3), indicating that the effect of PEG-G-CSF is specific.

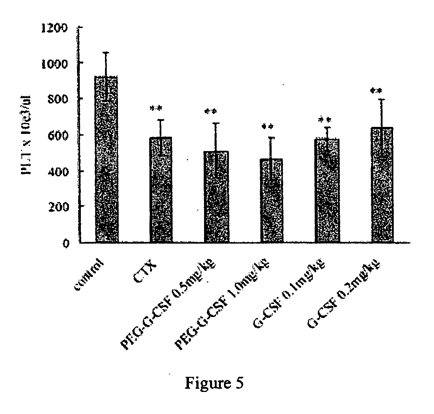

Continuous subcutaneous injection of G-CSF to the mice treated by cyclophosphamide also increases the peripheral white blood cell count. When 0.1 mg/kg is administered, the white blood cell count rises to normal level; when 0.2mg/kg is administered, the white blood cell count is higher than normal level (P <0.01 compared with the control, Figure 3), showing an obvious dose dependency. But G-CSF has no significant effect on peripheral blood erythrocyte, platelet count (Figure 4, 5).

According to the results, in the case of the total amount administered is equivalent from PEG-G-CSF and G-CSF, single subcutaneous injection of PEG-G-CSF and multiple subcutaneous injection of G-CSF have equivalent efficacy on peripheral blood white blood cell count of the mice.

### 4. Conclusions

PEG-G-CSF and G-CSF both significantly increase peripheral blood white blood cell count of the mice treated with cyclophosphamide. In the case of the total amount administered is equivalent, single subcutaneous injection of compound 1 of the present invention and multiple subcutaneous injection of G-CSF have equivalent efficacy on peripheral blood white blood cell count of the mice.

## Claims

1. A conjugate of general formula (II) or a pharmaceutical acceptable salt thereof: 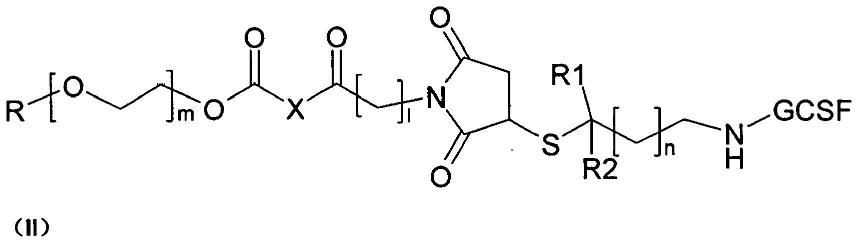 wherein,
R is selected from C₁₋₄ linear or branched alkyl, preferably methyl or ethyl, more preferably methyl;
R1 and R2 are each independently selected from hydrogen or C₁₋₄ linear or branched alkyl, preferably hydrogen, methyl or ethyl;
X is selected from O, S, NH, 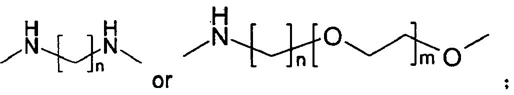
l is an integer selected from 1-20, preferably from 1-10, more preferably from 1-5;
m is an integer selected from 50-2500, preferably from 100-1000;
n is an integer selected from 1-20, preferably from 1-10, more preferably from 1-5;
G-CSF is selected from natural G-CSF, recombinant G-CSF or the product of gene mutation which has the function of G-CSF.

2. A conjugate or a pharmaceutical acceptable salt thereof of claim 1, wherein G-CSF is selected from G-CSF derivatives of a protein set forth in SEQ ID NO: 1-10.

3. A conjugate or a pharmaceutical acceptable salt thereof of claim 1, wherein the sequence of the said G-CSF is naturally occurring human G-CSF sequence.

4. A conjugate or a pharmaceutical acceptable salt thereof of claim 1, wherein the said G-CSF is Met-G-CSF shown in SEQ ID NO: 1.

5. A conjugate or a pharmaceutical salt thereof of claim 1, wherein the said conjugate includes: 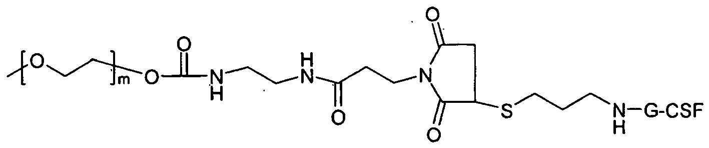 m is an integer selected from 400-500; or 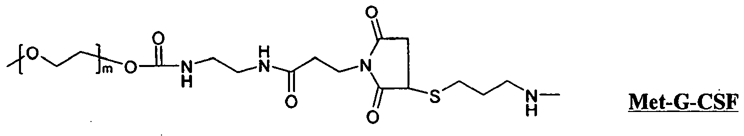 m is an integer selected from 400-500.

6. A conjugate or a pharmaceutical salt thereof of claim 1, wherein the said conjugate reacts with an acid to form a salt, wherein the acid is selected from organic or inorganic acids.

7. A conjugate or a pharmaceutical salt thereof of claim 6, wherein the said organic acid is selected from acetic acid, trifluoroacetic acid, propionic acid, butyric acid, maleic acid, or p-toluenesulfonic acid or mixture thereof, preferably acetic acid or trifluoroacetic acid; the inorganic acid is selected from hydrochloric acid, sulfuric acid, phosphoric acid or methanesulfonic acid or mixture thereof, preferably hydrochloric acid.

8. A method for preparing a conjugate of claim 1, comprising the following steps of:
1) reacting compounds of general formula (III) with N-terminal amino group of G-CSF by reductive amination to obtain compounds of general formula (IV): 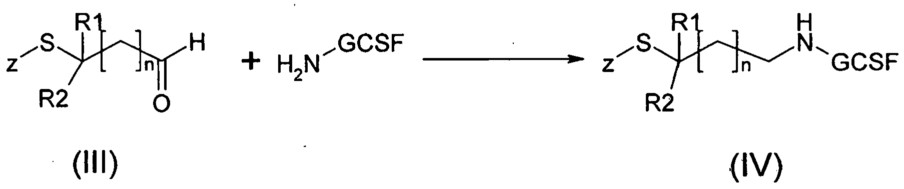
2) removing mercapto-protecting group from compounds of general formula (IV) to obtain compounds of general formula (V): 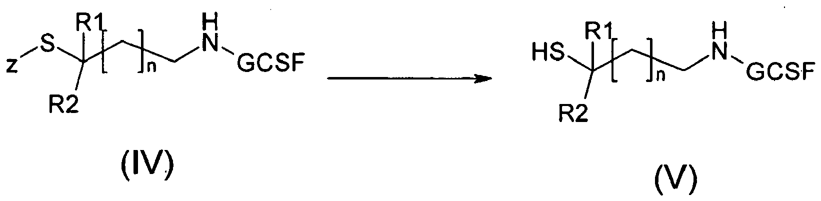
3) reacting compounds of general formula (V) with mPEG-MAL by Michael Addition Reaction to obtain compounds (II), 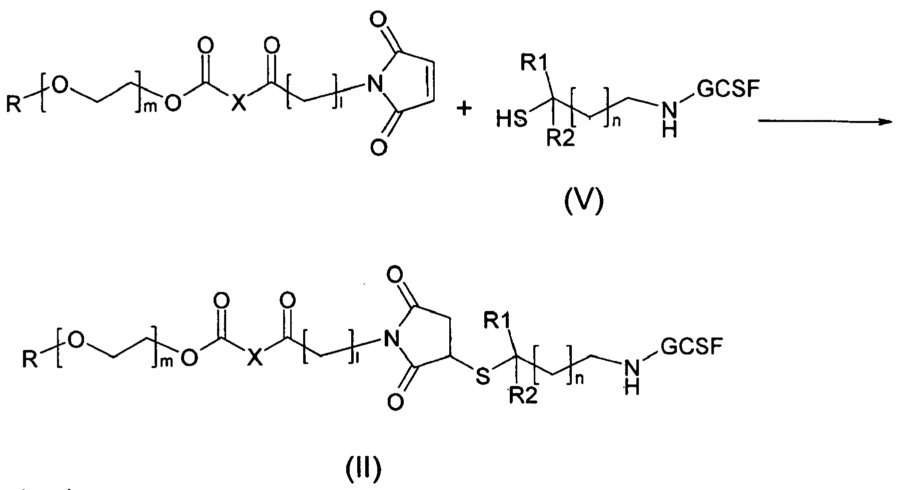 wherein,
R, R₁, R₂, G-CSF, X, l, m and n are as defined in claim 3; Z is mercapto-protecting group selected from formyl, acetyl, propionyl, trityl or tert-butyl group, preferably acetyl group.

9. The use of a conjugate or a pharmaceutical salt thereof of claim 1 in the preparation of the medicaments treating leucopenia caused by radiotherapy or chemotherapy, AIDS and other immune deficiency diseases, bacterial infections.

10. A conjugate or a pharmaceutical salt thereof of claim 1 for use in the treatment of leucopenia caused by radiotherapy or chemotherapy, AIDS and other immune deficiency diseases, bacterial infections.

11. A pharmaceutical composition, which comprises pharmaceutically effective amount of the conjugate or a pharmaceutical salt thereof of claim 1, and pharmaceutical acceptable carriers.

12. The use of the pharmaceutical composition of claim 10 in the preparation of the medicaments treating leucopenia caused by radiotherapy or chemotherapy, AIDS and other immune deficiency diseases, bacterial infections.

13. A pharmaceutical composition of claim 11 for use in the treatment of leucopenia caused by radiotherapy or chemotherapy, AIDS and other immune deficiency diseases, bacterial infections.

## Patentansprüche

1. Ein Konjugat der allgemeinen Formel (II) oder ein pharmazeutisch akzeptables Salz davon: 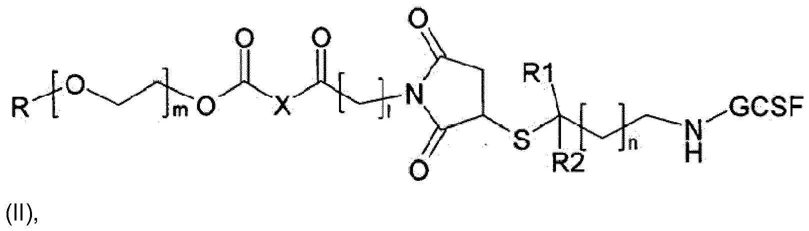 wobei
R aus linearem oder verzweigtem C₁₋₄-Alkyl, vorzugsweise Methyl oder Ethyl, mehr bevorzugt Methyl, ausgewählt ist;
R1 und R2 unabhängig voneinander aus Wasserstoff oder linearem oder verzweigtem C₁₋₄-Alkyl, vorzugsweise Wasserstoff, Methyl oder Ethyl, ausgewählt sind;
X aus O, S, NH, 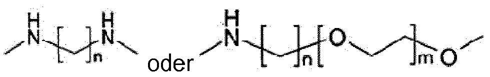 ausgewählt ist;
l eine ganze Zahl ausgewählt aus 1-20, vorzugsweise 1-10, mehr bevorzugt 1-5, ist;
m eine ganze Zahl ausgewählt aus 50-2500, vorzugsweise 100-1000, ist;
n eine ganze Zahl ausgewählt aus 1-20, vorzugsweise 1-10, mehr bevorzugt 1-5, ist;
G-CSF aus natürlichem G-CSF, rekombinantem G-CSF oder dem Produkt von Genmutation, das die Funktion von G-CSF hat, ausgewählt ist.

2. Ein Konjugat oder ein pharmazeutisch akzeptables Salz davon nach Anspruch 1, wobei G-CSF aus G-CSF-Derivaten eines Proteins, das in SEQ ID NO: 1-10 dargelegt ist, ausgewählt ist.

3. Ein Konjugat oder ein pharmazeutisch akzeptables Salz davon nach Anspruch 1, wobei die Sequenz des G-CSF natürlich auftretende humane G-CSF-Sequenz ist.

4. Ein Konjugat oder ein pharmazeutisch akzeptables Salz davon nach Anspruch 1, wobei das G-CSF Met-G-CSF gezeigt in SEQ ID NO: 1 ist.

5. Ein Konjugat oder ein pharmazeutisches Salz davon nach Anspruch 1, wobei das Konjugat umfasst: 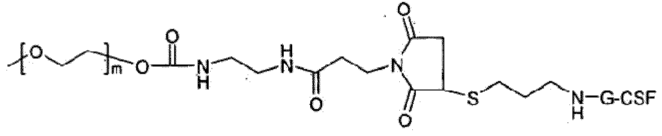 , wobei m eine ganze Zahl ausgewählt aus 400-500 ist; oder 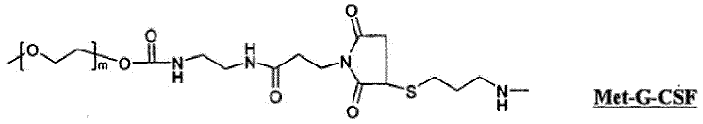 , wobei m eine ganze Zahl aus 400-500 ist.

6. Ein Konjugat oder ein pharmazeutisches Salz davon nach Anspruch 1, wobei das Konjugat mit einer Säure reagiert, um ein Salz auszubilden, wobei die Säure aus organischen oder anorganischen Säuren ausgewählt ist.

7. Ein Konjugat oder ein pharmazeutisches Salz davon nach Anspruch 6, wobei die organische Säure ausgewählt ist aus Essigsäure, Trifluoressigsäure, Propionsäure, Buttersäure, Maleinsäure oder p-Toluolsulfonsäure oder einer Mischung davon, vorzugsweise Essigsäure oder Trifluoressigsäure; die anorganische Säure ausgewählt ist aus Salzsäure, Schwefelsäure, Phosphorsäure oder Methansulfonsäure oder Mischungen davon, vorzugsweise Salzsäure.

8. Ein Verfahren zum Herstellen eines Konjugats nach Anspruch 1, das die folgenden Schritte aufweist:
1) Reagieren von Verbindungen der allgemeinen Formel (III) mit N-terminalen Aminogruppen von G-CSF durch reduktive Aminierung, um Verbindungen der allgemeinen Formel (IV) zu erhalten: 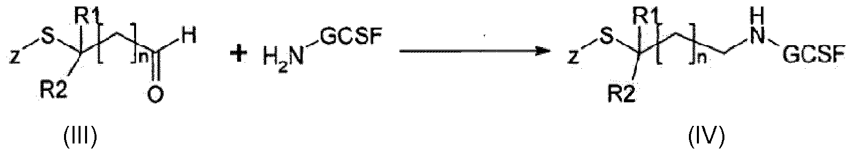
2) Entfernen der Mercapto-Schutzgruppe von Verbindungen der allgemeinen Formel (IV), um Verbindungen der allgemeinen Formel (V) zu erhalten: 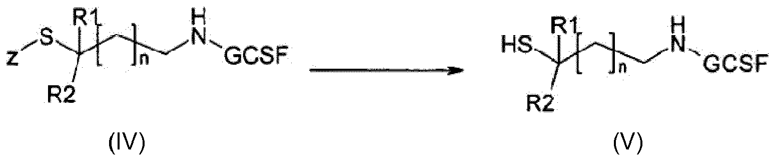
3) Reagieren von Verbindungen der allgemeinen Formel (V) mit mPEG-MAL durch Michael-Additionsreaktion, um Verbindungen (II) zu erhalten, 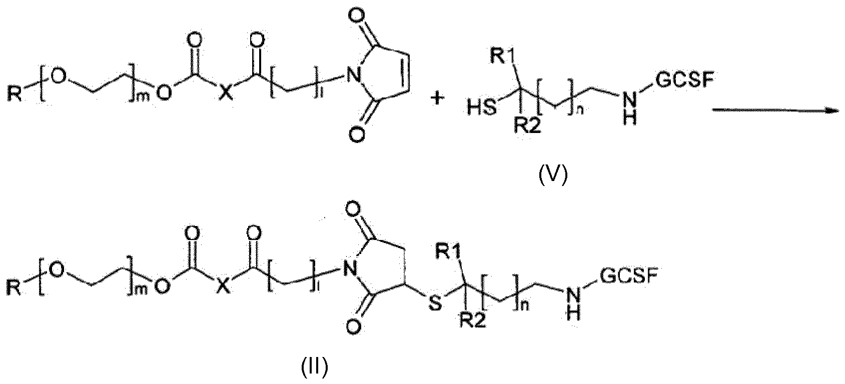 , wobei
R, R₁, R₂, G-CSF, X, l, m und n so wie in Anspruch 3 definiert sind; Z eine Mercapto-Schutzgruppe ausgewählt aus einer Formy-, Acetyl-, Propionyl-, Trityl- oder Tert-Butyl-Gruppe ist, vorzugsweise eine Acetyl-Gruppe.

9. Die Verwendung eines Konjugats oder eines pharmazeutischen Salzes davon nach Anspruch 1 bei der Herstellung der Medikamente, die Leukopenie verursacht durch Strahlentherapie oder Chemotherapie, AIDS und andere Immunschwächekrankheiten, Bakterieninfektionen behandeln.

10. Ein Konjugat oder ein pharmazeutisch Salz davon nach Anspruch 1 zur Verwendung bei der Behandlung von Leukopenie verursacht durch Strahlentherapie oder Chemotherapie, AIDS und andere Immunschwächekrankheiten, Bakterieninfektionen.

11. Eine pharmazeutische Zusammensetzung, die eine pharmazeutisch wirksame Menge des Konjugats oder eines pharmazeutischen Salzes davon nach Anspruch 1 und pharmazeutisch akzeptable Trägerstoffe enthält.

12. Die Verwendung einer pharmazeutischen Zusammensetzung nach Anspruch 10 bei der Herstellung der Medikamente, die Leukopenie verursacht durch Strahlentherapie oder Chemotherapie, AIDS und andere Immunschwächekrankheiten, Bakterieninfektionen behandeln.

13. Eine pharmazeutische Zusammensetzung nach Anspruch 11 zur Verwendung bei der Behandlung von Leukopenie verursacht durch Strahlentherapie oder Chemotherapie, AIDS und andere Immunschwächekrankheiten, Bakterieninfektionen.

## Revendications

1. Conjugué de formule générale (II) ou sel pharmaceutique acceptable de celui-ci : 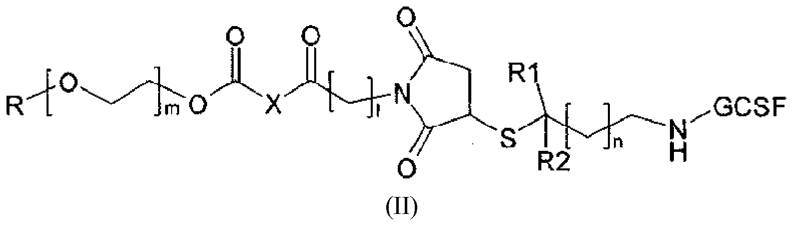 dans lequel,
R est choisi parmi un alkyle linéaire ou ramifié en C_{1 à 4}, de préférence un méthyle ou un éthyle, de manière davantage préférée un méthyle ;
R1 et R2 sont chacun indépendamment choisis parmi un hydrogène ou un alkyle linéaire ou ramifié en C_{1 à 4}, de préférence un hydrogène, un méthyle ou un éthyle ;
X est choisi parmi O, S, NH, 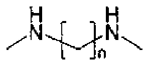 ou 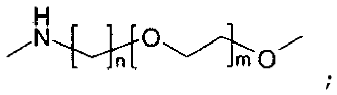
1 est un entier choisi parmi 1 à 20, de préférence parmi 1 à 10, de manière davantage préférée parmi 1 à 5 ;
m est un entier choisi parmi 50 à 2 500, de préférence parmi 100 à 1 000 ;
n est un entier choisi parmi 1 à 20, de préférence parmi 1 à 10, de manière davantage préférée parmi 1 à 5 ;
le G-CSF est choisi parmi un G-CSF naturel, un G-CSF recombinant ou le produit d'une mutation génique qui a la fonction de G-CSF.

2. Conjugué ou sel pharmaceutique acceptable de celui-ci selon la revendication 1, dans lequel le G-CSF est choisi parmi les dérivés de G-CSF d'une protéine décrite dans les SEQ ID NO : 1 à 10.

3. Conjugué ou sel pharmaceutique acceptable de celui-ci selon la revendication 1, dans lequel la séquence dudit G-CSF est la séquence du G-CSF humain présent à l'état naturel.

4. Conjugué ou sel pharmaceutique acceptable de celui-ci selon la revendication 1, dans lequel ledit G-CSF est le Met-G-CSF montré dans la SEQ ID NO : 1.

5. Conjugué ou sel pharmaceutique de celui-ci selon la revendication 1, dans lequel ledit conjugué inclut : 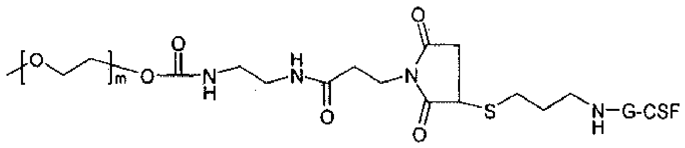 m est un entier choisi parmi 400 à 500 ; ou 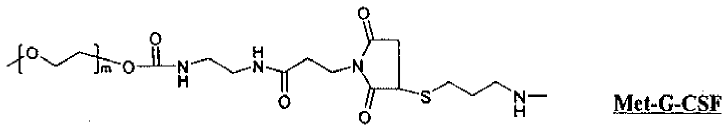 m est un entier choisi parmi 400 à 500.

6. Conjugué ou sel pharmaceutique de celui-ci selon la revendication 1, dans lequel ledit conjugué réagit avec un acide pour former un sel, dans lequel l'acide est choisi parmi les acides organiques ou inorganiques.

7. Conjugué ou sel pharmaceutique de celui-ci selon la revendication 6, dans lequel ledit acide organique est choisi parmi l'acide acétique, l'acide trifluoroacétique, l'acide propionique, l'acide butyrique, l'acide maléique, ou l'acide p-toluène sulfonique ou un mélange de ceux-ci, de préférence l'acide acétique ou l'acide trifluoroacétique ; l'acide inorganique est choisi parmi l'acide chlorhydrique, l'acide sulfurique, l'acide phosphorique ou l'acide méthanesulfonique ou un mélange de ceux-ci, de préférence l'acide chlorhydrique.

8. Procédé de préparation d'un conjugué de la revendication 1, comprenant les étapes suivantes :
1) la réaction de composés de formule générale (III) avec un groupe amino N-terminal d'un G-CSF par amination réductrice pour obtenir des composés de formule générale (IV) : 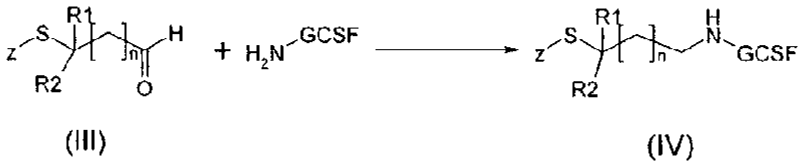
2) l'élimination d'un groupe protecteur de mercapto de composés de formule générale (IV) pour obtenir des composés de formule générale (V) : 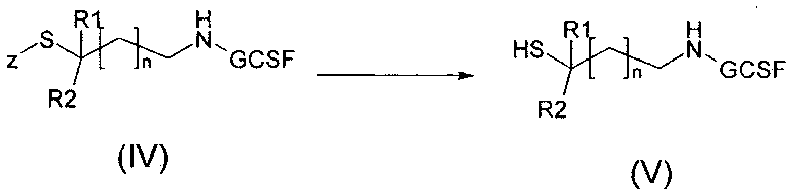
3) la réaction de composés de formule générale (V) avec mPEG-MAL par réaction d'addition de Michael pour obtenir des composés (II), 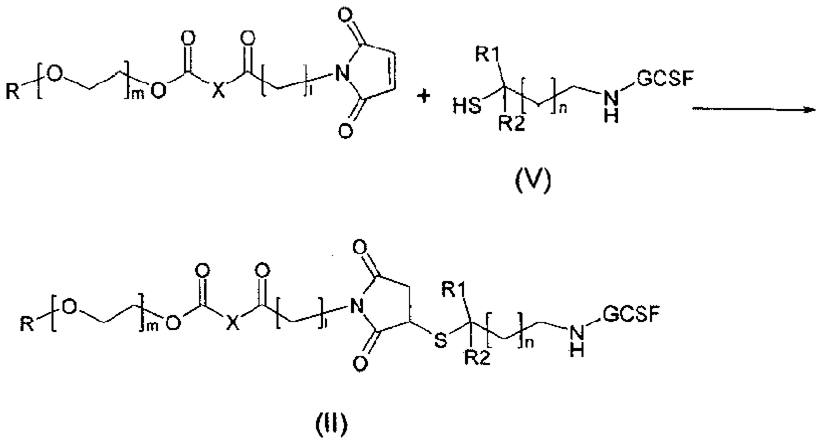 dans lequel,
R, R₁, R₂, G-CSF, X, 1, m et n sont comme définis dans la revendication 3 ; Z est un groupe protecteur de mercapto choisi parmi un groupe formyle, acétyle, propionyle, trityle ou tert-butyle, de préférence un groupe acétyle.

9. Utilisation d'un conjugué ou d'un sel pharmaceutique de celui-ci selon la revendication 1, dans la préparation de médicaments traitant une leucopénie provoquée par une radiothérapie ou une chimiothérapie, le SIDA et autres maladies d'immunodéficience, des infections bactériennes.

10. Conjugué ou sel pharmaceutique de celui-ci selon la revendication 1, pour une utilisation dans le traitement d'une leucopénie provoquée par une radiothérapie ou une chimiothérapie, le SIDA et autres maladies d'immunodéficience, des infections bactériennes.

11. Composition pharmaceutique, qui comprend une quantité pharmaceutiquement effective du conjugué ou d'un sel pharmaceutique de celui-ci de la revendication 1, et des vecteurs pharmaceutiques acceptables.

12. Utilisation de la composition pharmaceutique de la revendication 10 dans la préparation de médicaments traitant une leucopénie provoquée par une radiothérapie ou une chimiothérapie, le SIDA et autres maladies d'immunodéficience, des infections bactériennes.

13. Composition pharmaceutique selon la revendication 11, pour une utilisation dans le traitement d'une leucopénie provoquée par une radiothérapie ou une chimiothérapie, le SIDA et autres maladies d'immunodéficience, des infections bactériennes.
